# EUROPEAN PATENT APPLICATION

(11) **EP 1 884 228 A1**
(43) Date of publication of application: **06.02.2008**
(21) Application number: 06076514.6
(22) Date of filing: 01.08.2006
(51) Int. Cl.: A61K 8/06, A61K 8/41, A61K 8/36, A61K 8/26

(54) **Emulsion composition**

(71) Applicant: Sara Lee/DE N.V., 3532 AA Utrecht (NL)
(72) Inventor: Klomp, Andreas Johannes Anthonius, 2285 JE Rijswijk (NL); Pronk, Johannes, 2725 AM Zoetermeer (NL); Piessens, Josephus Petrus, 2275 BN Voorburg (NL); Voois, Karin, 2681 GC Monster (NL); Lambers, Johannes Wilhelmus Jacobus, 2564 LB Den Haag (NL)
(74) Representative: van Loon, C.J.J.

(57) **Abstract**

The invention provides an emulsion composition having a pH value in the range of from 4.0 to 7.0, which composition comprises a buffer, water, an oil in an amount in the range of from 3 to 20 wt%, a volatile nitrogen compound or salt thereof, and a thickener in an amount in the range of from 0.5 to 3 wt%, whereby all weights are based on total emulsion composition. The invention further provides a skin care composition and an aftershave composition comprising said emulsion composition. In addition, the invention relates to the use of said emulsion composition, skin care composition or aftershave composition for reducing and/or repairing skin damage that has been inflicted by means of a mechanical and/or chemical process, as well as to the use of said compositions for enhancing the skin barrier recovery, repair or restoration.

## Description

The present invention relates to an emulsion composition, a skin care composition and an aftershave composition comprising the emulsion composition, and the use of the emulsion composition, the skin care composition or the aftershave composition in reducing and/or repairing skin damage resulting from mechanical and/or chemical processes such as shaving, peeling and depilation.

It is well-appreciated that skin shaving brings about the removal of several layers of skin, resulting in loss of skin barrier function which leads to drying out of the skin and to less resistance to colonization and easier penetration of potentially harmful micro-organisms. Hence, from medical, cosmetic as well as hygienic perspective, a rapid recovery of the barrier function of the skin is required. It is further observed that the skin needs to have a proper moisture content in order to have an adequate barrier function.

Object of the present invention is to provide an emulsion composition which allows for a rapid repair of the skin barrier function after the skin has been damaged by way of a mechanical and/or chemical process, whereas in addition it adequately moistures the skin.

Surprisingly, it has now been found that this can be realized when use is made of a particular combination of emulsion components.

Accordingly, the present invention relates to an emulsion composition having a pH value in the range of from 4.0 to 7.0, which composition comprises a buffer, water, an oil in an amount in the range of from 3-20 wt%, a volatile nitrogen base or salt thereof, and a thickener in an amount in the range of from 0.5-3.0 wt%, whereby all weights are based on total emulsion composition.

The emulsion composition according to the present invention displays excellent skin barrier recovery and skin moisturizing properties, whereas in addition it is highly stable. Further, the application of the emulsion composition onto the skin gives an enjoyable refreshing cool feeling.

The thickener to be used in the emulsion composition according to the present invention is suitably chosen from the group consisting of mineral thickeners such as bentonite and montmorillite, natural and synthetic gums, synthetic polymers including co-polymers and cross-polymers, and any chemical modifications thereof. Examples of suitable natural or synthetic gums include gum arabic, gum tragacanth, cellulose gum, carraheenan, xanthan gum and their chemical modifications. Examples of suitable synthetic polymers include polyacrylate, dimethicon, vinyl, polyethylene, polypropylene glycol, and their chemical modifications.

Preferably, the thickener to be used in accordance with the present invention preferably comprises an anionic polymer comprising countercharges in the form of cationic groups that are derived from the volatile nitrogen base or salt thereof.

More preferably, the thickener is a polyacrylate or a copolymer or a cross-polymer of acrylate. Most preferably, the thickener is acryloyl di-methyltaurate/vinyl pyrrolidone copolymer.

The thickener to be used in accordance with the present invention is present in an amount in the range of from 0.5 to 3.0 wt%, based on total emulsion composition. Preferably, the thickener is present in an amount in the range of from 1.0 to 2.5 wt%, more preferably in an amount in the range of from 1.2 to 2.0 wt%, based on total emulsion composition.

The nitrogen base to be used is a volatile nitrogen base. In the context of the present invention this means that the nitrogen base has a boiling point below 200°C, preferably below 100°C, and more preferably below 65°C.

The volatile nitrogen base is preferably selected from the group consisting of ammonia, mono-ethanol amine, and alkyl amines of which the alkyl group comprises 1-4 carbon atoms. More preferably, the volatile nitrogen base is ammonia.

In accordance with the present invention the volatile nitrogen base can be present in the form of a salt. Suitable examples of such salts include, for instance, ammonium chloride, ammonium sulphate, ammonium lactate, and ammonium salts of the anionic polymer to be used as thickener as indicated hereinabove.

The volatile nitrogen base or its salt can suitably be present in an amount of from 0.02 to 0.5 mol per kg of the total emulsion composition.

Preferably, the volatile nitrogen base or its salt is present in an amount of from 0.05 to 0.25 mol per kg of the total emulsion composition.

The emulsion composition according to the present invention has a pH in the range of from 4.0 to 7.0. Preferably, the emulsion composition has a pH in the range of from 4.5 to 7.0. In a specially attractive embodiment of the present invention, in which low pH sensitive ingredients can advantageously be used, the emulsion has a pH in the range of from 5.0 to 6.5. It should be understood that these pH values are the pH values of the emulsion composition as such, i.e. before it is being applied to skin.

In the present emulsion composition a buffer is present. Suitably, said buffer has in the pH range of from 5.0 to 7.0 a buffer capacity which is less than 0.5 times the molarity of the volatile nitrogen base or salt thereof used, whereas in the pH range of from 3.8 to 5.0 the buffer has a buffer capacity which is at least equal to and at most twice as high as the molarity of the volatile nitrogen base or the salt thereof used.

Preferably, said buffer has in the pH range of from 5.0 to 7.0 a buffer capacity which is less than 0.2 times the molarity of the volatile nitrogen base or the salt thereof used. More preferably, a buffer capacity which is less than 0.1 times the molarity of the nitrogen base or the salt thereof used.

Suitable examples of buffers to be used in accordance with the present invention include those selected from the group of consisting of mono-, di-, and tri-carboxylic acids and their salts, and substituted mono-, di-, and tri-carboxylic acids and their salts.

Preferably, the buffer is lactic acid/lactate, succinic acid/succinate or citric acid/citrate. More preferably, the buffer is lactic acid/lactate.

In accordance with the present invention, the buffer is suitably present in an amount in the range of from 0.1 to 5.0 wt%, based on total emulsion composition. Preferably, the buffer is present in an amount in the range of from 0.5 to 2.5 wt%, based on total emulsion composition.

The amount of water in the emulsion composition according to the present invention can suitably be in the range of from 50-96 wt%, and is preferably in the range of from 65-85 wt%, based on total emulsion composition.

The particular combination of the components provides an emulsion composition that displays unique properties in terms of skin barrier recovery, skin moisturizing and a refreshing cool feel to the skin. Suitably, before application to skin the present emulsion composition has a pH in the range of from 5.0 to 6.5. After application of the emulsion composition to skin, and due to the evaporation of the volatile nitrogen base and water, the pH of the skin will attractively have a value in the range of from 4.0 to 5.0. The latter pH range will facilitate an optimal recovery of the skin barrier function. In other words, the emulsion composition according to the present invention brings advantageously the pH of the skin to a desired value.

The emulsion composition according to the present invention has a surprisingly attractive and beneficial effect on the barrier function of the skin in one or more ways. In particular, the present emulsion composition has been found to have a strengthening effect on at least one of the following functions: (i) the physico-chemical barrier realised by the keratinocytes and the epidermal lipids forming bi-layered sheets embedding those keratinocytes typically found in the deeper layers of the Stratum Corneum, (ii) the microbiological barrier (at the surface of the Stratum Corneum), and (iii) proper homeostasis in order to maintain a strong, smooth, unbroken and flexible skin condition (in particular in the superficial layers of the Stratum Corneum).

In the skin, a pH gradient exists from the skin surface to the deepest layers. The pH at the surface is almost always lower than in the deeper parts (such as Stratum Compactum and Stratum Granulosum) where pH reaches that of the internal body fluids.

Without being bound to any theory, it is believed that the positive effect of the present emulsion on the barrier function of the skin, is partially due to the capacity to restore and/or maintain the pH of different layers of the skin at a normal (i.e. non-pathological) value.

The present emulsion composition according to the invention thus has been found to have a balancing effect on the skin, which helps to maintain the skin integrity.

Yet another major advantage of the present invention is that no surfactant is required to provide a stable emulsion composition. Accordingly, the present emulsion composition is preferably in essence free of a surfactant.

Suitably, the present emulsion composition further comprises an astringent additive. Such an astringent additive tightens the skin. Suitably, the astringent additive is selected from the group consisting of metal salts, tannins and hazel extracts.

Preferably, the astringent additive is a soluble aluminium salt or a soluble zinc salt. More preferably, the astringent additive is alum or zinc sulphate.

The astringent additive is suitably present in an amount in the range of from 0.01 to 5.0 wt%, based on total emulsion composition. Preferably, the astringent additive is present in an amount in the range of from 0.1 to 1.0 wt%, based on total emulsion composition.

In addition, the emulsion composition can suitably comprise a fragrance. Any fragrance known to be used in body applications or aftershaves can be used for this purpose. The fragrance to be used is suitably present in an amount in the range of from 0.1-1.5 wt%, based on total emulsion composition. Preferably, the fragrance is present in an amount in the range of from 0.2 to 1.0 wt%, based on total emulsion composition.

Suitably, the emulsion composition in accordance with the present invention further comprises a humectant for promoting retention of moisture of the skin.

The humectant to be used in the present emulsion composition can suitably be selected from the group consisting of glycols, hydroxylated compounds, urea, pyrrolidine carboxylic acid, and any combinations thereof. Examples of suitable humectants include glycerol and sorbitol. Preferably, the humectant to be used in accordance with the present invention is glycerol.

Suitably, the humectant is present in an amount in the range of from 1.0-15.0 wt%, based on total emulsion composition. Preferably, the humectant is present in an amount in the range of from 2.0-6.0 wt%, based on total emulsion composition.

The oil to be used in the emulsion composition according to the present invention can suitably be selected from the group consisting of mineral and synthetic oils, vegetable oils and silicon-based oils.

The oil to be used is present in an amount in the range of from 3-20 wt%, based on total emulsion composition. Preferably, the oil is present in an amount in the range of from 5-20 wt%, and more preferably in an amount in the range of from 8-15 wt%, based on total emulsion composition.

In addition, the present emulsion composition may comprise any ingredients conventionally used in skin care compositions.

The emulsion composition according to the present invention can suitably be in the form of a cream, a lotion a paste or a gel. Preferably, the emulsion composition is present in the form of a lotion or cream, more preferably in the form of a lotion.

The emulsion composition can suitably have a viscosity in the range of from 5000-100000 cP (centipoises).

If applied in the form of a lotion, the emulsion composition will suitably have a viscosity in the range of from 5000-25000 cP, preferably in the range of 10000-20000 cP (as measured by Brookfield LVD, spindle 3, 6 rpm, 1 min., at 25°C).

If applied in the form of a cream, the emulsion composition will suitably have a viscosity in the range of from 25000-55000 cP, preferably in the range of 30000-45000 cP (as measured by Brookfield RVD, spindle 5, 5 rpm, 1 min., at 25°C).

The present invention also provides a skin care product comprising the emulsion composition according to the invention.

In addition, the present invention provides an aftershave composition which comprises the emulsion composition according to the present invention.

Further, the present invention relates to the use of the present emulsion composition, the skin care composition or the aftershave composition according to the present invention for reducing and/or repairing skin damage that has been inflicted by means of a mechanical process and/or chemical process. Examples of mechanical and/or chemical processes include shaving, peeling and depilation. It will be understood shaving and depilation can apply to any part of the body, including face, head, armpits, legs and the like, whereas peeling will usually only be applied to the face.

In addition, the present invention relates to the use of the emulsion composition, the skin care composition or the aftershave composition according to the present invention for enhancing the skin barrier recovery, repair or restoration.

### Example

### PREPARATION OF SKIN CARE COMPOSITION

A skin care composition in accordance with the present invention was prepared as follows. A first mixture was obtained by dissolving under stirring zinc sulphate (2.75 kg), glycerin (13.75 kg), caprylyl glycol (0.55 kg), lactic acid (0.55 kg), and sodium lactate (8.25 kg) in water (438.9 kg). A second mixture was obtained by mixing under stirring during 30 minutes ethylhexyl stearate (66.0 kg), a mixture of phenoxyethanol, ethyl paraben, methyl paraben and propyl paraben (5.5 kg in total), a perfume (2.75 kg) and ammonium acryloylmethyltaurate/vinyl pyrrolidone copolymer (11 kg). The second mixture was then added to the first mixture and the mixture so obtained was stirred for 20 minutes, whilst during 10 minutes a homogeniser was applied. In this way a cream was obtained.

### TESTING SKIN RECOVERY AND SKIN pH

The cream in accordance with the present invention was then used to help to repair the barrier of the tape-stripped skin of 10 subjects (individuals).

### TAPE-STRIP PROTOCOL

One spot (19x19 mm²) at the right forearm and one spot (19x19 mm²) at the left forearm were tape stripped with Pritt Sellotape Diamond ultra clear (Henkel) until Trans Epidermal Water Loss (TEWL) increase was in the range 15-25 g.m⁻².h⁻¹.
The following procedure was used:
- A piece of tape was applied at skin;
- A preheated stainless steel weight (1000g, 64g/cm²; T=34°C) was applied for five seconds at the taped skin;
- The tape was removed by jerking parallel to the skin (as fast as possible);
- This procedure was repeated 15-30 times; and
- The TEWL values (after five-minute waiting periods) were measured repeatedly to ensure that the increase of the TEWL values did not exceed 25 g.m⁻².h⁻¹.

### RECOVERY PERIOD

The barrier repair of the tape-stripped human skin was monitored during a period of nine days, both in respect of skin treated with the test product as well as untreated skin. During this period, the skin was soap-washed twice daily. This monitoring was done by measuring the TEWL values - values that are an excellent indicator for the quality of the skin's barrier.

### SOAP WASHING PROCEDURE

The inner sides of the forearms were washed after the tape stripping procedure on the evening of the first day, and subsequently on every morning and evening up to and including the morning of the ninth day. During the washing procedure the following protocol was used:
- The forearms were wetted with lukewarm tap water;
- During two minutes the forearms were washed by the subject with a 19% solution of natural soap bar (rubbing now and then);
- The forearms were then rinsed with tap water during about 15 seconds.
- The forearms were subsequently dried by means of dapping with a towel.

### PRODUCT APPLICATION PROTOCOL

The skin care composition in accordance with the present invention, and as described hereinbefore was applied at the tape-stripped skin by the subjects themselves. About 2 mg per cm² was applied every time. On the first day the composition was applied onto the skin after the last tape-stripping and after each soap washing, whereas on the second day up to and including the morning of the ninth day the composition was applied onto the skin after each soap washing.

### BIOPHYSICAL ASSESSMENTS

The TEWL values and skin pH values were then measured of the parts of skin treated as described hereinbefore. On the first day the values were measured before the treatment was started (baseline measurement), during the tape-stripping procedure (only TEWL values, data not shown), and 15 minutes after the soap washing treatment (start recovery measurement). Subsequently, the values were measured on the third, seventh and ninth day four hours after the skin care composition had been applied onto the skin. The TEWL values were measured by means of a Tewameter® TM300 (Courage + Khazaka), whereas the pH value of the skin was measured by means of a Skin-pH-Meter® PH 905 (Courage + Khazaka). The subjects were 10 healthy volunteers of either sex who were aged between 18 and 65 years. The following conditions were applied during the measurements: a temperature of 22° C (standard deviation of 1°C); a relative humidity of 45% (standard deviation of 5%); an equilibration period of 30 minutes. In the statistical analysis a two-tailed Paired Student's t-test was applied and a critical p-value (probability-value) for significance of 0.05 was used. In the data obtained the recovery is expressed as (1 - "TEWL increase" / "initial TEWL increase")*100%, whereas the TEWL increase is defined as the TEWL value of tape stripped skin minus the TEWL value of the intact skin. In other words, both the two tape-stripped skin fields as well as the intact (soap-washed) skin field were measured.

### RESULTS

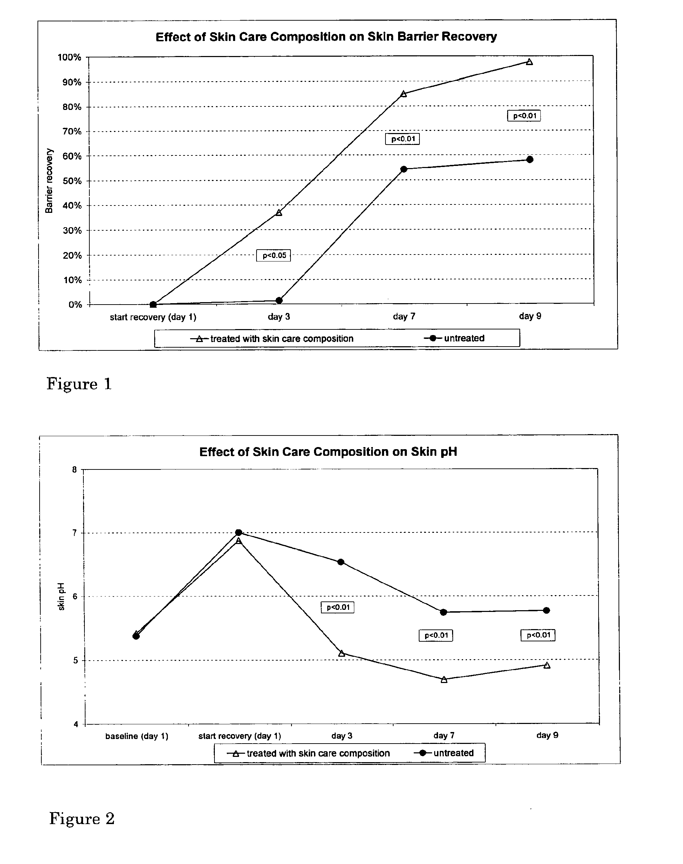

From the results shown in Figure 1, it will be clear that the application of the present skin care composition onto tape-stripped human skin resulted in a significantly quicker recovery of the barrier of the skin (see figure 1), when compared with untreated skin. In addition, the use of the composition in accordance with the present invention also resulted in a significant lower skin pH (see Figure 2).

## Claims

1. An emulsion composition having a pH value in the range of from 4.0 to 7.0, which composition comprises a buffer, water, an oil in an amount in the range of from 3 to 20 wt%, a volatile nitrogen compound or salt thereof, and a thickener in an amount in the range of from 0.5 to 3 wt%, whereby all weights are based on total emulsion composition.

2. The composition according to claim 1, wherein the thickener is selected from the group consisting of mineral thickeners such a bentonite and montmorillite, natural and synthetic gums, synthetic polymers, and any chemical modifications thereof.

3. The composition according to claim 1 or 2, wherein the thickener comprises an anionic polymer comprising countercharges in the form of cationic groups that are derived from the volatile nitrogen base or the salt thereof.

4. The composition according to claim 3, wherein the anionic polymer is a polyacrylate, or a co-polymer or cross-polymer of an acrylate.

5. The composition according to any one of claims 1-3, wherein the thickener is present in an amount in the range of from 1.0 to 2.5 wt%, based on total emulsion composition.

6. The composition according to any one of claims 1-5, wherein the volatile nitrogen base or salt thereof is present in an amount in the range of from 0.02 to 0.5 mol per kg of the total emulsion composition.

7. The composition according to any one of claims 1-6, wherein the volatile nitrogen base is selected from the group consisting of ammonia, mono-ethanol amine, and alkyl amines of which the alkyl group comprises 1-4 carbon atoms.

8. The composition according to claim 7, wherein the volatile nitrogen base is ammonia.

9. The composition according to any one of claims 1-8 having a pH in the range of from 5.0 to 6.5.

10. The composition according to any one of claims 1-9, wherein the buffer has in the pH range of from 5.0 to 7.0 a buffer capacity which is less than 0.5 times the molarity of the volatile nitrogen base or salt thereof, whereas in the pH range of from 3.8 to 5.0 the buffer has a buffer capacity which is at least equal to and at most twice as high as the molarity of the volatile nitrogen base or the salt thereof.

11. The composition according to claim 11, wherein the buffer has in the pH range of from 5.0 to 7.0 a buffer capacity which is less than 0.2 times the molarity of the volatile nitrogen base or the salt thereof.

12. The composition according to any one of claims 1-11, wherein the buffer is selected from the group of consisting of mono-, di-, and tri-carboxylic acids and their salts, and substituted mono-, di-, and tri-carboxylic acids and their salts.

13. The composition according to claim 12, wherein the buffer comprises lactic acid/lactate, succinic acid/succinate or citric acid/citrate.

14. The composition according to any one of claims 1-13, wherein the buffer is present in an amount in the range of from 0.1-5.0 wt%, based on total emulsion composition.

15. The composition according to any one of claims 1-14, wherein water is present in an amount in the range of from 50-96 wt%, based on total emulsion composition.

16. The composition according to any one of claims 1-15, wherein the composition further comprises an astringent additive.

17. The composition according to claim 16, wherein the astringent additive is selected from the group consisting of metal salts, tannins and hazel extracts.

18. The composition according to claim 17, wherein the astringent additive is a soluble aluminium salt or a soluble zinc salt.

19. The composition according to any one of claims 16-18, wherein the astringent additive is present in an amount in the range of from 0.01-5.0 wt%, based on total emulsion composition.

20. The composition according to any one of claims 1-19, wherein the composition further comprises a fragrance in an amount in the range of from 0.1-1.5 wt%, based on total emulsion composition.

21. The composition according to any one of claims 1-20, wherein the composition further comprises a humectant for promoting retention of moisture of the skin.

22. The composition according to claim 21, wherein the humectant is selected from the group consisting of glycols, hydroxylated compounds, urea, pyrrolidine carboxylic acid, and any combinations thereof.

23. The composition according to claim 22, wherein the humectant is glycerol.

24. The composition according to any one of claims 21-23, wherein the humectant is present in an amount in the range of from 1.0-15.0 wt%, based on total emulsion composition.

25. The composition according to any one of claims 1-24, wherein the composition is in the form of a lotion or a cream.

26. The composition according to claim 25, wherein the composition is in the form of a lotion having a viscosity in the range of from 5000-25000 cP (as measured by Brookfield LVD, spindle 3, 6 rpm, 1 min., at 25°C).

27. The composition according to claim 25, wherein the composition is in the form of a cream having a viscosity in the range of from 25000-55000 cP (as measured by Brookfield RVD, spindle 5, 5 rpm, 1 min., at 25°C).

28. A skin care composition comprising the emulsion composition as defined in any one of claims 1-27.

29. An aftershave composition comprising the emulsion composition as defined in any one of claims 1-27.

30. Use of a composition according to any one of claims 1-29 for reducing and/or repairing skin damage that has been inflicted by means of a mechanical and/or a chemical process.

31. Use of a composition according to any one of claims 1-29 for enhancing the skin barrier recovery, repair or restoration.
